# EUROPEAN PATENT APPLICATION

(11) **EP 3 605 547 A1**
(43) Date of publication of application: **05.02.2020**
(21) Application number: 19188324.8
(22) Date of filing: 25.07.2019
(51) Int. Cl.: G16H 20/60, G16H 20/30

(54) **COMPUTER-IMPLEMENTED METHOD AND INFORMATION PROCESSING DEVICE THAT GENERATE ADVICE INFORMATION CONCERNING DAILY HABIT IMPROVEMENT**

(30) Priority: 31.07.2018 JP 2018143571
(71) Applicant: SYSMEX CORPORATION, Kobe-shi Hyogo 651-0073 (JP)
(72) Inventor: Ohyama, Tomoko, Hyogo, 651-0073 (JP); Yoshinari, Hiromi, Hyogo, 651-0073 (JP); Watanabe, Yuuki, Hyogo, 651-0073 (JP); Ikeda, Koji, Hyogo, 651-0073 (JP); Suzuki, Kenichiro, Hyogo, 651-0073 (JP)
(74) Representative: Hoffmann Eitle

(57) **Abstract**

A computer-implemented method according to one or more aspects may include: analyzing action-related information on an action of a user to prepare an analysis result; and generating advice information concerning improvement of a daily habit of the user based on: the analysis result; and a piece of management policy information corresponding to a type of advice requested by the user among pieces of management policy information.

## Description

### BACKGROUND

The disclosure relates to a computer-implemented method and an information processing device that generate advice information concerning daily habit improvement.

Conventionally, efforts to improve daily habit by analyzing it have been made to maintain health. Improvement of the daily habit has been performed through improvement of the dietary habit and the exercise habit.

For example, International Publication No. WO 2007/072816 discloses a daily habit improvement support device that generates advice for improving the daily habit. This daily habit improvement support device generates advice for improving the daily habit in accordance with combination of a test result to be used for lifestyle-related disease diagnosis of a tested person and objective information objectively indicating the daily habit of the tested person, and provides the advice to the tested person.

To improve the daily habit, a user makes various efforts for improving the daily habit by exercising at a sport gym, receiving advice concerning the dietary habit from a dietary habit adviser, and receiving advice concerning health maintenance or disease prevention from a doctor at a clinic.

The purpose of improvement of the daily habit differs between a user who desires to acquire the exercise habit and a user who desires to improve the dietary habit, and thus advice information concerning daily habit improvement should differ as well. However, for example, when a service for improving the daily habit is provided to a user by using a technique disclosed in WO 2007/072816, advice is generated based on facts such as a test result used for lifestyle-related disease diagnosis and objective information objectively indicating the daily habit of the user, but does not reflect a subjective need of the user. For this reason, advice useful for the user may not be generated in some cases.

One or more aspects in the disclosure provide a computer-implemented method and an information processing device for generating advice information concerning daily habit improvement by reflecting a subjective need of the user, which allow a user to easily obtain the advice information that enables the user to continuously make life improvement.

### SUMMARY

A computer implemented method according to one or more aspects may include: analyzing action-related information on an action of a user to prepare an analysis result; and generating advice information concerning improvement of a daily habit of the user based on: the analysis result; and a piece of management policy information corresponding to a type of advice requested by the user among pieces of management policy information.

An information processing device according one or more aspects generates may include: an analysis unit that analyzes action-related information on an action of a user to prepare an analysis result; and an advice generation unit that generates advice information concerning improvement of a daily habit of the user based on: the analysis result; and a piece of management policy information corresponding to a type of advice requested by the user among pieces of management policy information.

### BRIEF DESCRIPTION OF DRAWINGS

FIG. 1 is a diagram illustrating a network configuration of an information processing device according to a first embodiment;
FIG. 2 is a diagram illustrating a functional block of an information processing device according to a first embodiment;
FIG. 3 is a diagram illustrating a physical configuration of an information processing device according to a first embodiment;
FIG. 4 is a flow diagram illustrating processing executed by an information processing device according to a first embodiment;
FIG. 5 is a diagram illustrating first exemplary management policy information referred to by an information processing device according to a first embodiment;
FIG. 6 is a diagram illustrating second exemplary management policy information referred to by an information processing device according to a first embodiment;
FIG. 7 is a diagram illustrating third exemplary management policy information referred to by an information processing device according to a first embodiment;
FIG. 8 is a diagram illustrating a functional block of an information processing device according to a second embodiment;
FIG. 9 is a diagram illustrating first exemplary management policy information referred to by an information processing device according to a second embodiment;
FIG. 10 is a diagram illustrating exemplary meal profile information stored in an information processing device according to a second embodiment;
FIG. 11 is a flow diagram illustrating processing executed by an information processing device according to a second embodiment;
FIG. 12 is a diagram illustrating an exemplary GUI provided to a user terminal by an information processing device according to a second embodiment;
FIG. 13 is a diagram illustrating an exemplary pie chart provided to a user terminal by an information processing device according to a second embodiment;
FIG. 14 is a diagram illustrating a functional block of an information processing device according to a third embodiment;
FIG. 15 is a diagram illustrating exemplary history information referred to by an information processing device according to a third embodiment;
FIG. 16 is a flow diagram illustrating processing executed by an information processing device according to a third embodiment;
FIG. 17 is a diagram illustrating exemplary advice information generated by an information processing device according to a third embodiment;
FIG. 18 is a diagram illustrating exemplary set target values referred to by an information processing device according to a third embodiment;
FIG. 19 is a diagram illustrating a first exemplary GUI provided to a user terminal by an information processing device according to a third embodiment; and
FIG. 20 is a diagram illustrating a second exemplary GUI provided to a user terminal by an information processing device according to a third embodiment.

### DETAILED DESCRIPTION

A computer-implemented method of generating advice information concerning daily habit improvement according to one or more aspects includes: analyzing action-related information on an action of a user; and generating advice information concerning improvement of a daily habit of the user based on an analysis result of the action-related information and a piece of management policy information corresponding to a type of advice requested by the user among pieces of management policy information.

According to one or more aspects, advice information to which a subjective need of the user is reflected can be generated by generating advice information concerning daily habit improvement based on an analysis result of the action-related information on the action of the user and a piece of management policy information corresponding to a type of advice requested by the user among pieces of management policy information. Accordingly, the user can easily obtain advice information that enables the user to continuously make life improvement that satisfies the own need.

In one or more aspects, the advice information may be generated based on the analysis result and the piece of management policy information selected from among the pieces of management policy information.

According to one or more aspects, information suitable for the analysis result of the action-related information on the action of the user can be selected from among the pieces of management policy information, and thus advice more suitable for the living condition of the user can be provided.

In one or more aspects, the advice information may be generated based on the analysis result and the piece of management policy information selected based on predetermined identification information.

According to one or more aspects, since the piece of management policy information is selected by using the predetermined identification information, the piece of management policy information suitable for the action of the user can be selected when the advice information is provided.

In one or more aspects, a characteristic of the daily habit of the user may be extracted based on the action-related information, and the advice information may be generated based on the extracted characteristic and the piece of management policy information.

According to one or more aspects, since the advice information is generated based on the characteristic of the daily habit of the user, the advice information customized for the daily habit of the user can be provided.

In one or more aspects, the piece of management policy information may be adjusted based on a profile of the user.

According to one or more aspects, since the piece of management policy information is adjusted based on the profile of the user, advice information customized in accordance with the characteristic of the user can be provided.

In one or more aspects, the advice information may be generated in response to a request from an action management service provider configured to provide a service that manages an action related to a daily habit to the user, based on the analysis result of the action-related information and the piece of management policy information corresponding to the action management service provider, and the generated advice information may be provided to the action management service provider having requested advice provision.

According to one or more aspects, since the advice information concerning daily habit improvement is provided based on the analysis result of the action-related information on the action of the user and the piece of management policy information corresponding to the action management service provider having requested advice provision, advice information customized for each action management service provider can be provided. Accordingly, investment cost of each action management service provider can be reduced, and the user can easily obtain advice that enables the user to continuously make life improvement through the action management service provider.

In one or more aspects, the piece of management policy information corresponding to the action management service provider having requested advice provision may be selected from among the pieces of management policy information based on information for selecting the corresponding piece of management policy information.

According to one or more aspects, since the information for selecting the piece of management policy information corresponding to the action management service provider having requested advice provision is used to select the piece of management policy information, management policy information suitable for the action management service provider can be selected, and advice information more suitable for a request from the action management service provider can be provided.

In one or more aspects, the advice information may be generated based on the analysis result and the piece of management policy information selected based on identification information of the action management service provider.

According to one or more aspects, since the piece of management policy information is selected based on the identification information of the action management service provider, the piece of management policy information suitable for the action management service provider can be selected, and advice information more suitable for a request from the action management service provider can be provided.

In one or more aspects, task information supporting the user having received the service by the action management service provider may be generated.

According to one or more aspects, since the task information is generated, a task to be performed by the user for daily habit improvement can be clarified.

In one or more aspects, new advice information concerning improvement of the daily habit may be generated based on temporal change of an action of the user having received the service by the action management service provider and the piece of management policy information corresponding to the action management service provider having requested advice provision.

According to one or more aspects, since the new advice information is generated in accordance with the change of the action of the user having received the service by the action management service provider, new advice more suitable for the user can be provided based on the effect of the previous advice.

In one or more aspects, the new advice information may be generated when the action of the user having received the service by the action management service provider does not satisfy a criterion requested by the advice information.

According to one or more aspects, since the new advice information is generated when the action of the user having received the service by the action management service provider does not satisfy the requested criterion, new advice more suitable for the user can be provided based on insufficiency of the effect of the previous advice.

In one or more aspects, a plurality of action management service providers may provide a service that manages an action related to a daily habit to respective users.

According to one or more aspects, customized advice information can be provided to the action management service providers that provide the service to the respective users.

In one or more aspects, two or more different pieces of the advice information corresponding to two or more of the action management service providers having requested advice provision may be generated based on the analysis result of the action-related information and two or more of the pieces of management policy information corresponding to the two or more action management service providers.

According to one or more aspects, when the analysis result of the action-related information is identical, advice information customized for each action management service provider can be provided based on the two or more pieces of management policy information corresponding to the two or more action management service providers.

In one or more aspects, the action-related information may include at least one of a meal image obtained by capturing a meal of the user, vital data of the user, and information indicating the amount of exercise of the user.

According to one or more aspects, advice information customized for each action management service provider can be provided based on an analysis result obtained by analyzing the action of the user based on objective data and the piece of management policy information corresponding to the action management service provider.

In one or more aspects, the advice information may be provided to computers 2A and 2B communicate with applications 10A and 10B installed on terminals 1A and 1B corresponding to users.

According to one or more aspects, since the advice information is provided to each computer that communicates with an application installed on a terminal for a user, the advice information can be notified to the terminal for the user through the computer.

In one or more aspects, the advice information may be provided through an application program interface (API) to computers 2A and 2B that communicate with applications 10A and 10B installed on terminals 1A and 1B corresponding to users.

According to one or more aspects, since the advice information is provided through the API, each action management service provider can easily use the advice information, which leads to reduction of investment cost.

An information processing device 3 according to one or more aspects generates advice information concerning a daily habit. The information processing device 3 includes: an analysis unit 30 that analyzes action-related information on an action of a user; and an advice generation unit 31 that generates advice information concerning improvement of a daily habit of the user based on an analysis result of the action-related information and a piece of management policy information corresponding to a type of advice requested by the user among pieces of management policy information.

According to one or more aspects, advice information to which a subjective need of the user is reflected can be generated by generating advice information concerning daily habit improvement based on an analysis result of the action-related information on the action of the user and the piece of management policy information corresponding to a type of advice requested by the user among the pieces of management policy information. Accordingly, the user can easily obtain advice information that enables the user to continuously make life improvement that satisfies the own need.

In one or more aspects, the advice generation unit 31 may generate the advice information based on the analysis result and the piece of management policy information selected from among the pieces of management policy information.

According to one or more aspects, information suitable for the analysis result of the action-related information on the action of the user can be selected from among the pieces of management policy information, and thus advice more suitable for the living condition of the user can be provided.

In one or more aspects, the advice generation unit 31 may generate the advice information based on the analysis result and the piece of management policy information selected based on predetermined identification information.

According to one or more aspects, since the piece of management policy information is selected by using the predetermined identification information, the piece of management policy information suitable for the action of the user can be selected when the advice information is provided.

In one or more aspects, the analysis unit 30 may extract a characteristic of the daily habit of the user based on the action-related information, and the advice generation unit 31 may generate the advice information based on the extracted characteristic and the piece of management policy information.

According to one or more aspects, since the advice information is generated based on the characteristic of the daily habit of the user, the advice information customized for the daily habit of the user can be provided.

In one or more aspects, the advice generation unit 31 may adjust the piece of management policy information based on a profile of the user.

According to one or more aspects, since the piece of management policy information is adjusted based on the profile of the user, advice information customized in accordance with the characteristic of the user can be provided.

In one or more aspects, the advice generation unit may generate the advice information based on the analysis result and the piece of management policy information selected based on identification information of the action management service provider.

According to one or more aspects, since the advice information concerning daily habit improvement is provided based on the analysis result of the action-related information on the action of the user and the piece of management policy information corresponding to the action management service provider having requested advice provision, advice information customized for each action management service provider can be provided. Accordingly, investment cost of each action management service provider can be reduced, and the user can easily obtain advice that enables the user to continuously make life improvement through the action management service provider.

In one or more aspects, the advice generation unit 31 may select the piece of management policy information corresponding to the action management service provider having requested advice provision from among the pieces of management policy information based on information for selecting the corresponding management policy information.

According to one or more aspects, since the information for selecting the piece of management policy information corresponding to the action management service provider having requested advice provision is used to select the piece of management policy information, the piece of management policy information suitable for the action management service provider can be selected, and advice information more suitable for a request from the action management service provider can be provided.

In one or more aspects, the advice generation unit 31 may generate the advice information based on the analysis result and the management policy information selected based on identification information of the action management service provider.

According to one or more aspects, since the piece of management policy information is selected based on the identification information of the action management service provider, the piece of management policy information suitable for the action management service provider can be selected, and advice information more suitable for a request from the action management service provider can be provided.

In one or more aspects, the advice generation unit 31 may generate task information supporting the user having received the service by the action management service provider.

According to one or more aspects, since the task information is generated, a task to be performed by the user for daily habit improvement can be clarified.

In one or more aspects, the advice generation unit 31 may generate the new advice information based on temporal change of an action of the user having received the service by the action management service provider and the management policy information corresponding to the action management service provider having requested advice provision.

According to one or more aspects, since the new advice information is generated in accordance with the change of the action of the user having received the service by the action management service provider, new advice more suitable for the user can be provided based on the effect of the previous advice.

In one or more aspects, the advice generation unit 31 may generate the new advice information when the action of the user having received the service by the action management service provider does not satisfy a criterion requested by the advice information.

According to one or more aspects, since the new advice information is generated when the action of the user having received the service by the action management service provider does not satisfy the requested criterion, new advice more suitable for the user can be provided based on insufficiency of the effect of the previous advice.

In one or more aspects, a plurality of action management service providers may provide a service that manages an action related to a daily habit to respective users.

According to one or more aspects, advice information customized for action management service providers that provide the service to respective users can be provided.

In one or more aspects, the advice generation unit 31 may generate two or more different pieces of the advice information corresponding to two or more of the action management service providers having requested advice provision based on the analysis result of the action-related information and two or more of the pieces of management policy information corresponding to the two or more action management service providers.

According to one or more aspects, when the analysis result of the action-related information is identical, advice information customized for each action management service provider can be provided based on the two or more pieces of management policy information corresponding to the two or more action management service providers.

In one or more aspects, the action-related information may include at least one of a meal image obtained by capturing a meal of the user, vital data of the user, and information indicating the amount of exercise of the user.

According to one or more aspects, advice information customized for each action management service provider can be provided based on an analysis result obtained by analyzing the action of the user based on objective data and the piece of management policy information corresponding to the action management service provider.

One or more aspects may further include a communication unit 30d that provides the advice information to computers 2A and 2B that communicate with applications 10A and 10B installed on terminals 1A and 1B corresponding to users.

According to one or more aspects, since the advice information is provided to each computer that communicates with an application installed on a terminal for a user, the advice information can be notified to the terminal for the user through the computer.

One or more aspects may further include a communication unit 10d that provides the advice information through an application program interface (API) to computers 2A and 2B that communicate with applications 10A and 10B installed on terminals 1A and 1B corresponding to users.

According to one or more aspects, since the advice information is provided through the API, each action management service provider can easily use the advice information, which leads to reduction of investment cost.

In a method of generating advice information concerning daily habit improvement according to one or more aspects, information communication terminals 1A and 1B of users communicate with an information processing device 3 that analyzes a daily habit; the information communication terminals 1A and 1B receive, from the information processing device 3 target values of tasks related to daily habit improvement and produced by the information processing device 3 based on a piece of management policy information corresponding to a type of advice requested by the users among pieces of management policy information; and the information communication terminals 1A and 1B display a graphical user interface (GUI) including the progress of each task and the ratio of the target value of each task to the sum of the target values of the tasks.

According to one or more aspects, since each information processing device produces the target values of tasks related to daily habit improvement in accordance with a subjective need of the user based on management policy information corresponding to a type of advice requested by the user among pieces of management policy information, and the information communication terminal of the user displays a GUI including the progress of each task and the ratio of the target value of each task to the sum of the target values, the user can understand the progress and ratio of each task at a glance.

In one or more aspects, the ratio of the target value of each task to the sum of the target values of the tasks may be displayed in a pie chart.

According to one or more aspects, since the ratio of the target value of each task is displayed in a pie chart, the ratio of the target value of each task to the sum of the target values of the tasks can be understood at a glance.

In one or more aspects, the ratio of the target value of each task to the sum of the target values of the tasks may be displayed in the arc length of a slice in a pie chart.

According to one or more aspects, since the ratio of the target value of each task is displayed in the arc length of a slice in a pie chart, the ratio of the target value of each task to the sum of the target values of the tasks can be understand as the ratio of the length of the arc at a glance.

In one or more aspects, the ratio of the target value of each task to the sum of the target values of the tasks may be displayed in the arc length of a slice in a pie chart, and the progress of each task may be displayed in the width of the pie chart in a diametrical direction.

According to one or more aspects, since the ratio of the target value of each task is displayed in the arc length of a slice in a pie chart, the ratio of the target value of each task to the sum of the target values of the tasks can be understood as the ratio of the length of the arc at a glance. In addition, since the progress of each task is displayed in the width of the pie chart in the diametrical direction, the progress difference between the tasks can be understood at a glance.

In one or more aspects, the target values of the tasks may be displayed in a circle concentric with the pie chart.

According to one or more aspects, the target values of the tasks can be understood at a glance while the ratio of the target value of each task to the sum of the target values of the tasks is indicated in the pie chart.

In one or more aspects, the current total value of any of the tasks may be displayed in the length of an arc concentric with the pie chart.

According to one or more aspects, the current total value of any of the tasks can be understood at a glance while the ratio of the target value of each task to the sum of the target values of the tasks is indicated in the pie chart.

In one or more aspects, the arc concentric with the pie chart may be displayed in a manner different between a case where the sum of the current values of the tasks is smaller than the sum of the target values of the tasks and a case where the sum of the current values of the tasks is larger than the sum of the target values of the tasks.

According to one or more aspects, whether the sum of the current values of the tasks is larger than the sum of the target values can be understood at a glance.

In one or more aspects, the information processing device 3 may communicate with servers 2A and 2B of action management service providers each configured to provide a service that manages an action related to a daily habit of the user, the information communication terminals 1A and 1B may receive, from servers 2A and 2B, target values of tasks related to daily habit improvement produced by the information processing device 3 based on a piece of management policy information corresponding to each action management service provider, and the information communication terminals 1A and 1B may display a graphical user interface (GUI) including the progress of each task and the ratio of the target value of each task to the sum of the target values of the tasks.

According to one or more aspects, since the information processing device produces the target values of tasks related to daily habit improvement based on the management policy information corresponding to each action management service provider and the information communication terminal of the user displays a GUI including the progress of each task and the ratio of the target value of each task to the sum of the target values, the user can understand the progress and ratio of each task at a glance.

One or more aspects provide a method and an information processing device that generate advice information that is related to daily habit improvement and provides, in accordance with a piece of management policy information of each action management service provider, advice for performing life improvement continuously executable by a user so that the user can easily obtain the advice for performing the life improvement.

The following describes embodiments (hereinafter referred to as "an embodiment" or one or more embodiments) according to one or more aspects with reference to the accompanying drawings. In the drawings, components denoted by an identical reference sign have identical or similar configurations.

### [First embodiment]

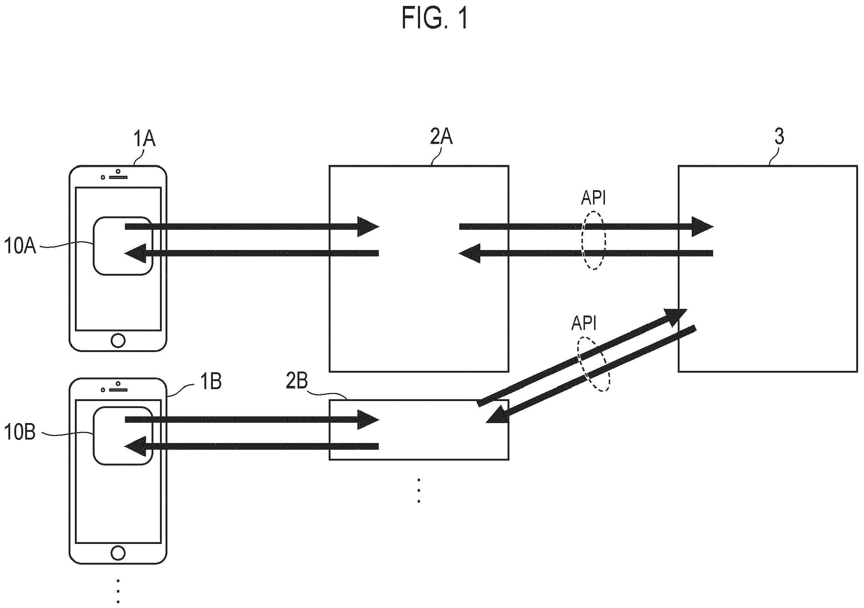

FIG. 1 is a diagram illustrating a network configuration of an information processing device 3 according to a first embodiment. The information processing device 3 provides advice information concerning daily habit improvement to an action management service provider configured to provide a service that manages, improves, or supports an action related to a daily habit to the user. The drawing exemplarily illustrates a first server 2A corresponding to a first action management service provider and a second server 2B corresponding to a second action management service provider, and illustrates a case where the information processing device 3 communicates with the first server 2A and the second server 2B. A user receives provision of the service that manages an action related to a daily habit from the action management service provider. The drawing exemplarily illustrates a first user terminal 1A corresponding to a first user and a second user terminal 1B corresponding to a second user, and illustrates a case where the first server 2A and the first user terminal 1A communicate with each other and the second server 2B and the second user terminal 1B communicate with each other. The action related to a daily habit means an action included in the habit of the user, and includes, for example, exercise, smoking, drinking, sleep, and meal. The action management service provider provides to the user, for example, a service that manages an exercise habit to the user, or a service that manages the content of a meal.

The action management service provider is an individual or a company who provides the service that manages an action related to a daily habit to the user. The action management service provider is, for example, a training gym, a personal trainer, a business operator who provides a health management service, or a doctor. The information processing device 3 is used by a business operator (hereinafter referred to as an advice provider) who provides advice information concerning improvement of the daily habit of the user to the action management service provider. The advice provider provides the advice information to servers (the first server 2A and the second server 2B) corresponding to the action management service providers by using the information processing device 3. The action management service providers provide the service that manages an action related to the daily habit to respective users. Improvement of the daily habit of each user is improvement of an action related to the daily habit, and specifically, includes increase of the amount of exercise, reduction of the amount of smoking, reduction of the amount of drinking, a constant sleep duration, a constant meal time, three meals per day, improvement of meal nutritional balance, and the like.

The action management service providers manage an action related to the daily habit of the user with management policies different from each other. The information processing device 3 refers to management policy information corresponding to each action management service provider, and provides advice information in accordance with the management policy information of the action management service provider. The management policy information is information as collection of policies based on which the action management service provider manages an action related to the daily habit of the user, and is set for each action management service provider and different between the action management service providers. For example, when each action management service provider provides a service that manages the exercise habit of the user, the service provided to the user differs between the action management service providers as a training gym and a doctor. Specifically, the training gym provides the service that manages the exercise habit of the user from a viewpoint of body fitness, and the doctor provides the service that manages the exercise habit of the user from a viewpoint of health maintenance or disease prevention. The information processing device 3 includes the management policy information to which such a difference in action management purpose between the action management service providers is reflected. However, the action management service providers may use the same management policy information. The advice information is information indicating a target to which the user improves the daily habit, and includes information related to a task to be performed by the user, and information related to action not to be performed by the user. Provision of the advice information to an action management service provider means transfer of the advice information to the action management service provider as an individual or a company. Provision of the advice information may be performed by transmitting electronic data representing the advice information to a server corresponding to the action management service provider through a communication network, or may be performed by mailing a printed sheet on which the advice information is written to the action management service provider as an individual or a company.

The first user receives a service from the first server 2A corresponding to the first action management service provider through a first application 10A of the first user terminal 1A. Similarly, the second user receives a service from the second server 2B corresponding to the second action management service provider through a second application 10B of the second user terminal 1 B. The first application 10A and the second application 10B are, for example, applications provided from the action management service providers. The application 10A may be a general-purpose application such as a web browser, not a dedicated application provided from the action management service provider.

A communication unit of the information processing device 3 provides advice information to a computer that communicates with an application installed on a terminal for a user. Specifically, the communication unit of the information processing device 3 provides advice information to the first server 2A that communicates with the first application 10A installed on the first user terminal 1A corresponding to the first user. The communication unit of the information processing device 3 also provides advice information to the second server 2B that communicates with the second application 10B installed on the second user terminal 1B corresponding to the second user. In this manner, since the advice information is provided to each computer that communicates with an application installed on a terminal for a user, the advice information can be notified to the terminal for the user through the computer.

Alternatively, the communication unit of the information processing device 3 provides advice information through an application program interface (API) to a computer that communicates with an application installed on a user terminal for a user. Specifically, the communication unit of the information processing device 3 provides advice information through the API to the first server 2A that communicates with the first application 10A installed on the first user terminal 1A corresponding to the first user. More specifically, a hypertext transfer protocol (HTTP) request for provision of the advice information in a format defined by the API is transmitted from the first server 2A to the information processing device 3, and in response to the request, the advice information is provided as a HTTP response from the information processing device 3 to the first server 2A. The communication unit of the information processing device 3 also provides advice information through the API to the second server 2B that communicates with the second application 10B installed on the second user terminal 1B corresponding to the second user. In this manner, since advice information is provided through the API, each action management service provider can easily use the advice information, which leads to reduction of investment cost.

An embodiment describes below a case where the first server 2A and the second server 2B are separated from the information processing device 3, the first server 2A may be integrated with the information processing device 3. In addition, the second server 2B may be integrated with the information processing device 3.

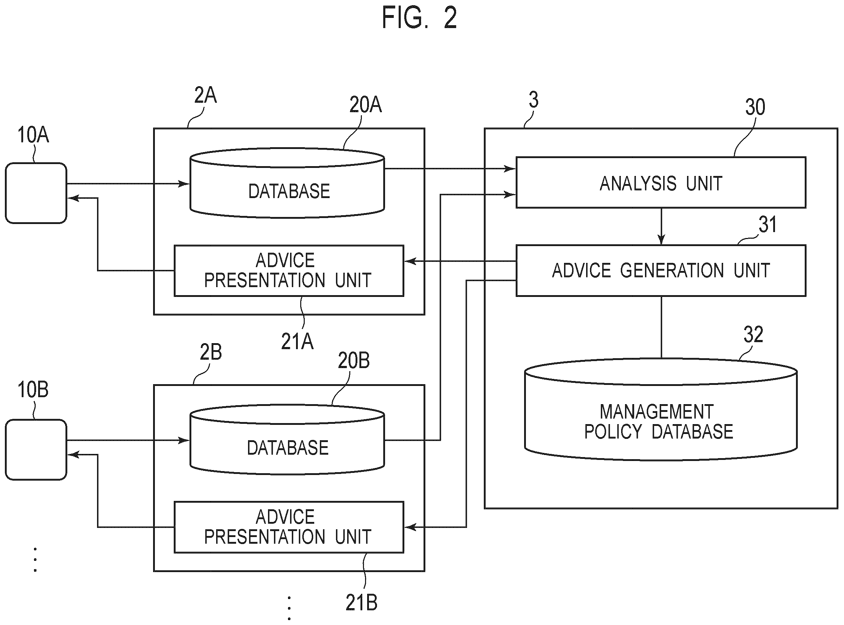

FIG. 2 is a diagram illustrating a functional block of the information processing device 3 according to a first embodiment. The information processing device 3 includes an analysis unit 30, an advice generation unit 31, and a management policy database 32. As illustrated in the next drawing, the information processing device 3 also includes a communication unit 10d that provides generated advice information to an action management service provider having requested advice provision. The advice provision request from the action management service provider to the advice provider is performed by electronic data transmission, oral instruction, or mailing instruction. The first server 2A and the second server 2B may each request, through a predetermined API, the information processing device 3 for provision of advice information concerning daily habit improvement.

The analysis unit 30 analyzes action-related information on the action of a user. The action-related information is information directly or indirectly indicating the action of the user, and includes, for example, a meal image obtained by capturing a meal of the user, vital data of the user, and information indicating the amount of exercise of the user. The meal image is information directly indicating the diet action of the user. The vital data varies with the awake state and emotion of the user, is information indirectly indicating the sleep or exercise action of the user, and includes, for example, heart rate, blood pressure, respiratory rate, body temperature, brain wave, and the like. The amount of exercise of the user is information directly indicating the exercise action of the user. The data format of the action-related information is a text related to the action of the user or an image related to the action of the user. The analysis unit 30 analyzes the action-related information, and extracts a characteristic related to generation of improvement advice to the user. The first server 2A receives the action-related information corresponding to an action related to the daily habit from the first application 10A corresponding to the first user, and stores the action-related information in a database 20A. Then, the first server 2A provides the action-related information of the first user stored in the database 20A to the analysis unit 30. Similarly, the second server 2B receives the action-related information corresponding to an action related to the daily habit from the second application 10B corresponding to the second user, and stores the action-related information in a database 20B. Then, the second server 2B provides the action-related information of the second user stored in the database 20B to the analysis unit 30. A result of the analysis of the action-related information by the analysis unit 30 includes information obtained by quantifying the daily habit of the user. The analysis result of the action-related information includes information obtained by quantifying, for example, exercise duration, sleep duration, sleep onset time, wake-up time, meal time, and nutrition included in a meal of the user.

The advice generation unit 31 generates advice information concerning improvement of the daily habit of the user based on the analysis result of the action-related information and a piece of management policy information corresponding to a type of advice requested by the user among pieces of management policy information. The type of advice requested by the user is based on a subjective need of the user. The advice may include, for example, advice for acquiring the exercise habit, advice for improving the dietary habit, advice for maintaining health, and advice for preventing disease. The piece of management policy information corresponding to the type of advice requested by the user is a piece of management policy information suitable for fulfilling the subjective need of the user. When receiving service provision from an action management service provider such as a sport gym or a doctor, the user is thought to select the action management service provider expected to provide the type of advice requested by the user. Specifically, it is normal that: the user selects a sport gym as the action management service provider when the user intends to acquire the exercise habit; the user selects a dietary habit adviser as the action management service provider when the user intends to improve the dietary habit; or the user selects a doctor as the action management service provider when the user intends to maintain health or prevent disease. Thus, the advice generation unit 31 may generate advice information concerning daily habit improvement based on the analysis result of the action-related information and the piece of management policy information corresponding to the action management service provider having requested advice provision. In such a case, advice information in accordance with the subjective need of the user is generated. The advice generation unit 31 generates advice information concerning improvement of the daily habit of the user based on a daily habit characteristic extracted from the action-related information by the analysis unit 30 and the piece of management policy information stored in the management policy database 32. The daily habit characteristic is a matter representing the daily habit and is information based on which whether the daily habit is desirable is determined. The daily habit characteristic indicates, for example, whether the user has three meals per day, nutritional balance, sleep duration, wake-up time, the amount of regular exercise, and the like. Accordingly, since the advice information is generated based on the characteristic of the daily habit of the user, advice information customized for the daily habit of the user can be provided. The advice generation unit 31 generates two or more different pieces of advice information corresponding to two or more action management service providers having requested advice provision based on the analysis result of the action-related information and two or more pieces of management policy information corresponding to the two or more action management service providers. Accordingly, when the analysis result of the action-related information is identical, advice information customized for each action management service provider can be provided based on two or more different pieces of management policy information corresponding to two or more action management service providers. For example, when the analysis result of the action-related information of the user includes identical information related to the amount of exercise, advice information generated based on a piece of management policy information related to body fitness includes an exercise plan for developing muscle at a particular site, and advice information generated based on a piece of management policy information related to health maintenance or disease prevention includes the target value of the number of steps per day.

The advice generation unit 31 provides the generated advice information to the first server 2A or the second server 2B corresponding to an action management service provider having requested advice provision. For example, when having received the advice information provision, the first server 2A provides the advice information to the first application 10A through an advice presentation unit 21A. Similarly, when having received the advice information provision, the second server 2B provides the advice information to the second application 10B through an advice presentation unit 21B.

In the information processing device 3 according to an embodiment, since advice information concerning daily habit improvement is provided based on the analysis result of action-related information on the action of the user and the piece of management policy information corresponding to an action management service provider having requested advice provision, advice information customized for each action management service provider can be provided. Accordingly, investment cost of each action management service provider can be reduced, and the user can easily obtain advice that enables the user to continuously make life improvement through the action management service provider.

Although an embodiment describes the example in which advice information is provided from the information processing device 3 to the first server 2A or the second server 2B of an action management service provider and provided to the first user terminal 1A or the second user terminal 1B through the action management service provider, the advice information may be directly provided from the information processing device 3 to the first user terminal 1A or the second user terminal 1B.

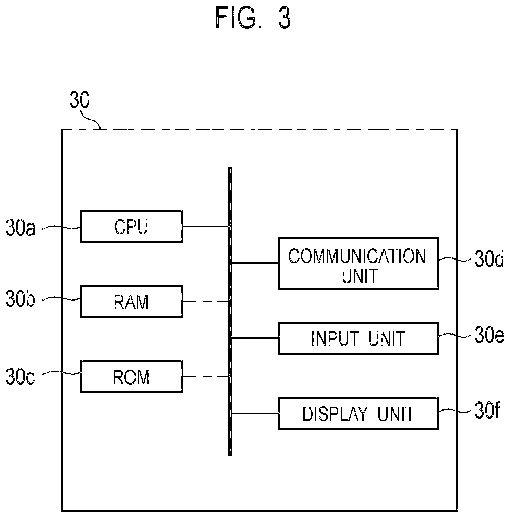

FIG. 3 is a diagram illustrating a physical configuration of the information processing device 3 according to an embodiment. The information processing device 3 includes a central processing unit (CPU) 30a corresponding to a calculation unit, a random access memory (RAM) 30b corresponding to a memory, a read only memory (ROM) 30c corresponding to a memory, a communication unit 30d, an input unit 30e, and a display unit 30f. These components are connected with each other through a bus to achieve mutual data transmission and reception. The present example describes below a case where the information processing device 3 includes one computer, but the information processing device 3 may be achieved by combining a plurality of computers. The configuration illustrated in FIG. 3 is merely exemplary, and the information processing device 3 may include a component other than these components or may not include some of these components.

The CPU 30a is a controller that performs control and data computation and processing related to execution of a computer program stored in the RAM 30b or the ROM 30c. The CPU 30a is a calculation unit that executes a computer program (advice information generation program) that generates advice information concerning daily habit improvement. The CPU 30a receives various kinds of data from the input unit 30e and the communication unit 30d, and displays a result of computation of the data on the display unit 30f or stores the result in the RAM 30b and the ROM 30c.

The RAM 30b is a data rewritable memory and includes, for example, a semiconductor storage element. The RAM 30b stores data such as the advice information generation program executed by the CPU 30a and management policy information. Such data is merely exemplary, and the RAM 30b may store data other than such data or may not store part of the data.

The ROM 30c is a data readable memory and includes, for example, a semiconductor storage element. The ROM 30c stores, for example, the advice information generation program and data not to be overwritten.

The communication unit 30d is an interface through which the information processing device 3 is connected with another instrument such as the first server 2A or the second server 2B. The communication unit 30d is connected with a communication network such as the Internet.

The input unit 30e receives data input from the user and includes, for example, a keyboard and a touch panel.

The display unit 30f visually displays a result of computation by the CPU 30a, and is achieved by, for example, a liquid crystal display (LCD). The display unit 30f displays, for example, the generated advice information.

The advice information generation program may be provided being stored in a computer-readable storage medium such as the RAM 30b or the ROM 30c or may be provided through the communication network connected through the communication unit 30d. In the information processing device 3, the CPU 30a executes the advice information generation program to achieve various operations of the analysis unit 30, the advice generation unit 31, and the like described with reference to FIG. 2. These physical components are merely exemplary and do not necessarily need to be independent components. For example, the information processing device 3 may include a large-scale integration (LSI) obtained by integrating the CPU 30a, the RAM 30b, and the ROM 30c.

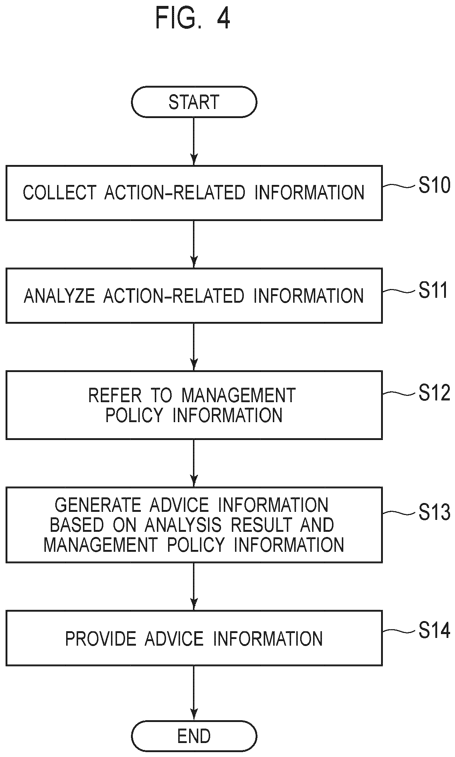

FIG. 4 is a flow diagram of processing executed by the information processing device 3 according to a first embodiment. First, the information processing device 3 collects action-related information from the first server 2A and the second server 2B (S10), and analyzes the action-related information (S11).

Thereafter, the information processing device 3 refers to management policy information stored in the management policy database 32 (S12), and generates advice information based on an analysis result of action-related information and the management policy information (S13).

Lastly, the information processing device 3 provides the generated advice information to an action management service provider having requested provision of the advice information (S14).

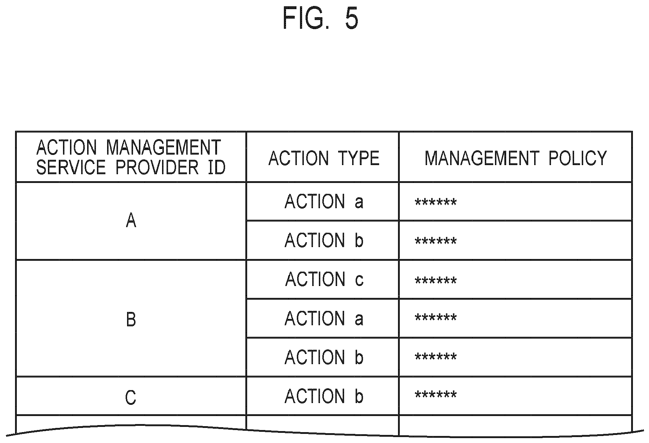

FIG. 5 is a diagram illustrating first exemplary management policy information referred to by the information processing device 3 according to a first embodiment. In the present example, the management policy database 32 stores management policy information for identification information (action management service provider ID) of each action management service provider. The advice generation unit 31 generates advice information based on management policy information selected based on the analysis result of the action-related information and predetermined identification information. Since management policy information is selected by using the predetermined identification information, management policy information suitable for the action of the user can be selected when the advice information is provided. More specifically, in the present example, the advice generation unit 31 generates the advice information based on the analysis result of the action-related information and the management policy information selected based on the identification information of the action management service provider. Since the management policy information is selected based on the identification information of the action management service provider, management policy information suitable for the action management service provider can be selected, and advice information more suitable for a request from the action management service provider can be provided.

In the present example, the management policy database 32 stores management policy information or a management policy for each user action to be managed. Specifically, the management policy database 32 stores management policy information or a management policy for each "action type". The action type is, for example, "action a", "action b", or "action c", and specifically, is an optional action type related to the daily habit, such as "exercise", "walking", "jogging", "meal", "carbohydrate ingestion", "protein ingestion", "fat ingestion", "salt ingestion", "dietary fiber ingestion", "alcohol ingestion", "smoking", or "sleep". These action types are exemplary actions related to the daily habit. The analysis result of the action-related information of the user indicates to which of these action types an action of the user corresponds.

The advice generation unit 31 generates advice information based on the analysis result of the action-related information and a piece of management policy information selected from among pieces of the management policy information. Accordingly, information suitable for the analysis result of the action-related information on the action of the user can be selected from among the pieces of the management policy information, and thus advice more suitable for the living condition of the user can be provided. The advice generation unit 31 selects the management policy information corresponding to the action management service provider having requested advice provision from among the pieces of the management policy information based on information for selecting the corresponding management policy information. The information for selecting the management policy information corresponding to the action management service provider having requested advice provision is, for example, the action management service provider ID or the action type. Since the information for selecting the management policy information corresponding to the action management service provider having requested advice provision is used to select the management policy information, management policy information suitable for the action management service provider can be selected, and advice information more suitable for a request from the action management service provider can be provided.

The management policy information is, for example, "Increase total calorie consumption: standard value [X kcal/day or more, Y kcal/week or more, Z kcal/month or more]", "Limit total calorie count of meals: standard value [X kcal/day or less, Y kcal/week or less, Z kcal/month or less]", "Limit carbohydrate ingestion: standard value [X g/day or less, Y g/week or less, Z g/month or less]", "Increase the amount of protein ingestion: standard value [X g/day or more, Y g/week or more, Z g/month or more]", "Limit fat ingestion: standard value [X g/day or less, Y g/week or less, Z g/month or less]", "Limit salt ingestion: standard value [X g/day or less, Y g/week or less, Z g/month or less]", "Increase the amount of dietary fiber ingestion: standard value [X g/day or more, Y g/week or more, Z g/month or more]", "Limit alcohol ingestion: standard value [X ml/day or less, Y ml/week or less, Z ml/month or less]", "Limit the number of cigarettes smoked: standard value [X /day or less]", or "Increase sleep duration: standard value [X hours/day or more]". "X", "Y", and "Z" represent optional numerical values. A standard value included in the management policy information is set for each action management service provider or for each user.

The advice generation unit 31 generates advice information such as "Have exercise so that the total calorie consumption per day is X kcal or more", "Limit the total calorie count of meals to X kcal or less", "Reduce carbohydrate ingestion", "Increase protein ingestion", "Reduce fat ingestion", "Reduce salt ingestion", "Increase dietary fiber ingestion", "Reduce cigarette smoking", or "Have appropriate sleep duration".

The advice generation unit 31 generates task information supporting the user having received service by the action management service provider. The task information is information related to a task to be performed by the user, and is, for example, "Walk for one hour or longer per day", "Jog for 30 minutes or longer per day", "Limit the amount of carbohydrate ingestion per day to X g or less", "Increase the amount of protein ingestion per meal to X g or more", or "Limit the amount of alcohol ingestion per week to Y ml or less". The task information may be generated by the advice presentation unit 21A of the first server 2A or the advice presentation unit 21B of the second server 2B based on advice information provided from the information processing device 3, and may be provided to the user. Since the task information is generated, a task to be performed by the user for daily habit improvement can be clarified.

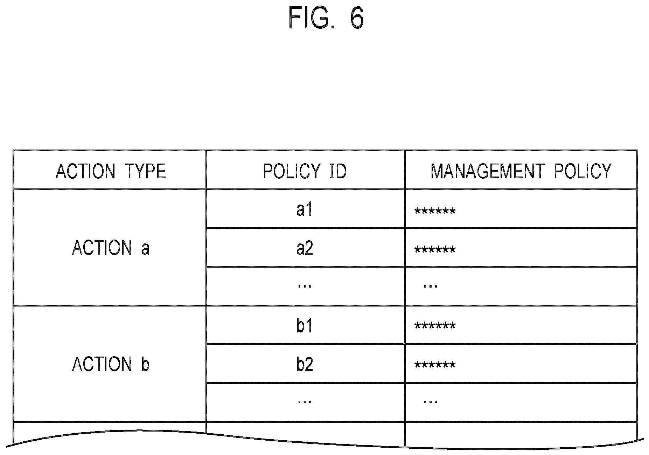

FIG. 6 is a diagram illustrating second exemplary management policy information referred to by the information processing device 3 according to a first embodiment. In the present example, the management policy database 32 stores management policy information for each action type. For example, management policy information having "policy ID" of "a1" and management policy information having "policy ID" of "a2" are stored for "action type a". Similarly, management policy information having "policy ID" of "b1" and management policy information having "policy ID" of "b2" are stored for "action type b".

The advice generation unit 31 generates advice information based on an analysis result of action-related information and management policy information selected based on predetermined identification information. The predetermined identification information is information for uniquely specifying the management policy information and is, for example, a policy ID. Since the management policy information is selected by using the predetermined identification information, management policy information suitable for the action of the user can be selected when the advice information is provided. The predetermined identification information may be an optional combination of strings and numerical values.

When requesting provision of advice information to the information processing device 3, the first server 2A and the second server 2B corresponding to action management service providers transmit identification information (policy ID) of management policy information or a management policy determined for each action type to be managed to the information processing device 3. Then, the information processing device 3 selects management policy information to be used to generate advice information based on the received identification information (policy ID).

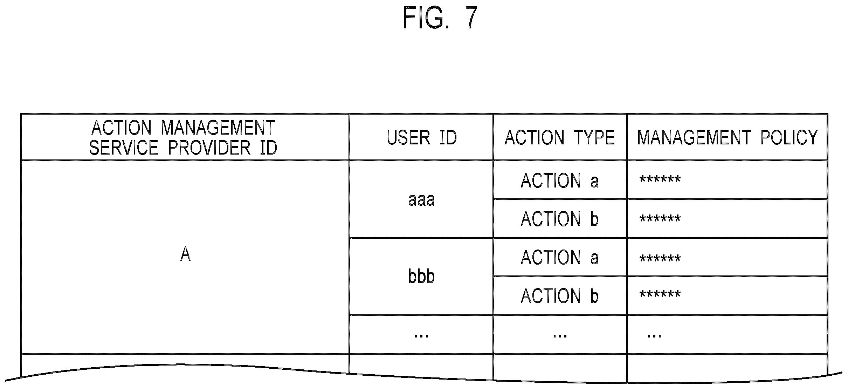

FIG. 7 is a diagram illustrating third exemplary management policy information referred to by the information processing device 3 according to a first embodiment. In the present example, the management policy database 32 stores management policy information for the identification information (action management service provider ID) of each action management service provider. The management policy database 32 also stores management policy information for each user ID as user identification information. For example, management policy information having "action type" of "action a" and management policy information having "action type" of "action b" are stored for user ID "aaa". Similarly, management policy information having "action type" of "action a" and management policy information having "action type" of "action b" are stored for user ID "bbb". In this manner, since management policy information is stored for each user ID, each action management service provider can set management policy information in accordance with the characteristic of a user.

The advice generation unit 31 adjusts management policy information based on the profile of a user. The profile of a user is a biography of the user and includes, for example, the age, height, weight, sex, past medical history, and the like of the user. For example, the advice generation unit 31 adjusts a standard value included in the management policy information based on the profile of the user specified by a user ID. More specifically, when the management policy information is "Increase total calorie consumption: standard value [X kcal/day or more, Y kcal/week or more, Z kcal/month or more]", the advice generation unit 31 adjusts to increase the total calorie consumption as the age of the user is younger, adjusts so that the total calorie consumption is appropriate for the height and weight of the user, adjusts so that the total calorie consumption for a male are larger than the total calorie consumption for a female depending on the sex of the user, or adjusts to reduce the total calorie consumption in accordance with the past medical history of the user. In this manner, since the management policy information is adjusted based on the profile of the user, advice information customized in accordance with the characteristic of the user can be provided.

### [Second embodiment]

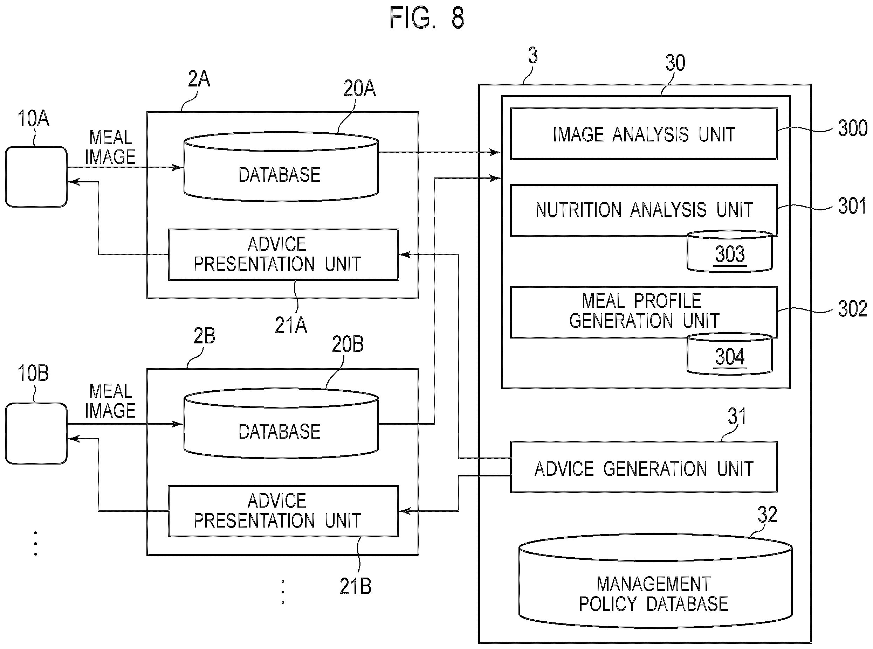

FIG. 8 is a diagram illustrating a functional block of the information processing device 3 according to a second embodiment. Similarly to the information processing device 3 according to a first embodiment, the information processing device 3 according to a second embodiment includes the analysis unit 30, the advice generation unit 31, and the management policy database 32, but is different from the information processing device 3 according to a first embodiment in that the analysis unit 30 includes an image analysis unit 300, a nutrition analysis unit 301, a meal profile generation unit 302, a nutrition database 303, and a meal profile database 304. In an embodiment, the first user and the second user use the first user terminal 1A and the second user terminal 1B to transmit meal images obtained by capturing meals to the first server 2A and the second server 2B, respectively. Each meal image is, for example, an image captured before a user has a meal so that all dishes are included in the image capturing range. The first server 2A receives the meal image as action-related information from the first application 10A corresponding to the first user, and stores the meal image in the database 20A. Then, the first server 2A provides the meal image of the first user stored in the database 20A to the analysis unit 30. Similarly, the second server 2B receives the meal image as action-related information from the second application 10B corresponding to the second user, and stores the meal image in the database 20B. Then, the second server 2B provides the action-related information of the second user stored in the database 20B to the analysis unit 30.

The image analysis unit 300 analyzes the meal image captured by each user, and analyzes the type of the meal. The type of the meal is, for example, information indicating the menu and amount of the meal. The image analysis unit 300 analyzes the meal image through, for example, an analysis algorithm using machine learning or deep learning. The image analysis unit 300 identifies the type of the meal included in the meal image by using, for example, a neural network such as a convolutional neural network (CNN).

The nutrition analysis unit 301 refers to the nutrition database 303 to analyze nutrition included in the meal type identified by the image analysis unit 300. The nutrition includes, for example, the amounts of carbohydrate, fat, protein, and dietary fiber. The nutrition database 303 stores nutrition, the contained amount of nutrition, and the total calorie count for each meal type. The nutrition analysis unit 301 refers to the nutrition database 303 to specify nutrition corresponding to the meal type, and outputs the nutrition and the total calorie count included in the meal.

The meal profile generation unit 302 generates meal profile information as information indicating the dieting tendency of each user. The meal profile generation unit 302 generates, as the meal profile information, for example, information accumulating, in a temporally sequential manner, the total calorie count of a meal taken by a user and the amount of ingestion of each nutrition thereof. The meal profile information is an exemplary result of analysis by the analysis unit 30.

The advice generation unit 31 generates advice information concerning daily habit improvement based on the meal profile information and management policy information. The advice generation unit 31 generates new advice information based on temporal change of an action of the user having received a service by an action management service provider and management policy information corresponding to an action management service provider having requested advice provision. For example, when a dieting action against the management policy information has continued for a predetermined duration, the advice generation unit 31 generates the advice information and notifies the advice information to the server (the first server 2A or the second server 2B) of the corresponding action management service provider. The case where the dieting action against the management policy information has continued for a predetermined duration is, for example, a case where the amount of predetermined nutrition ingestion against the management policy information has continued for a predetermined duration. In this manner, since the new advice information is generated in accordance with the change of the action of the user having received a service by the action management service provider, new advice more suitable for the user can be provided based on the effect of the previous advice.

The advice generation unit 31 generates new advice when the action of a user having received a service by an action management service provider does not satisfy a criterion requested by advice information. For example, when a dieting action not satisfying a criterion requested by management policy information has continued for a predetermined duration, the advice generation unit 31 lowers the requested criterion and generates new advice information. Since new advice information is generated when the action of a user having received a service by an action management service provider does not satisfy a requested criterion, new advice more suitable for the user can be provided based on insufficiency of the effect of the previous advice.

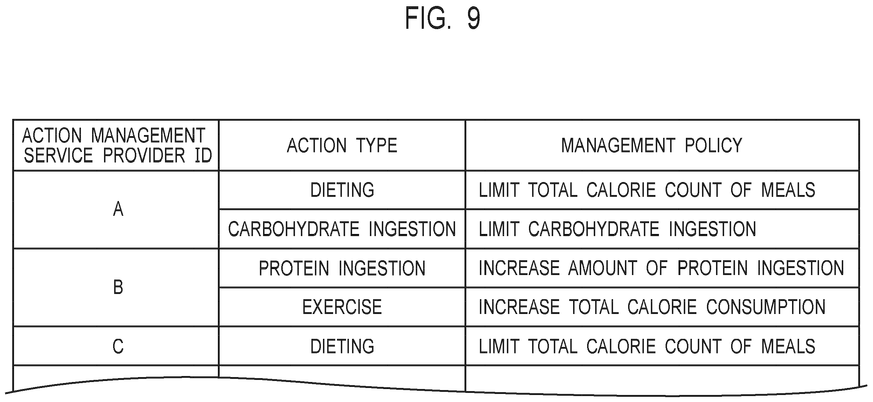

FIG. 9 is a diagram illustrating first exemplary management policy information referred to by the information processing device 3 according to a second embodiment. In the present example, the management policy database 32 stores management policy information for the identification information (action management service provider ID) of each action management service provider. The management policy database 32 also stores management policy information corresponding to an action of the user to be managed. Specifically, the management policy database 32 stores management policy information or a management policy for each "action type". The action type is, for example, "meal", "carbohydrate ingestion", "protein ingestion", or "exercise". These are merely exemplary, and the action type may be the type of an optional action.

The management policy information is "Limit the total calorie count of meals" when the action type is "meal", "Limit carbohydrate ingestion" when the action type is "carbohydrate ingestion", "Increase the amount of protein ingestion" when the action type is "protein ingestion", or "Increase the total calorie consumption" when the action type is "exercise". In this manner, since the action type is set for each action management service provider and the management policy information is set for each action type, advice information more suitable for a request from an action management service provider can be provided.

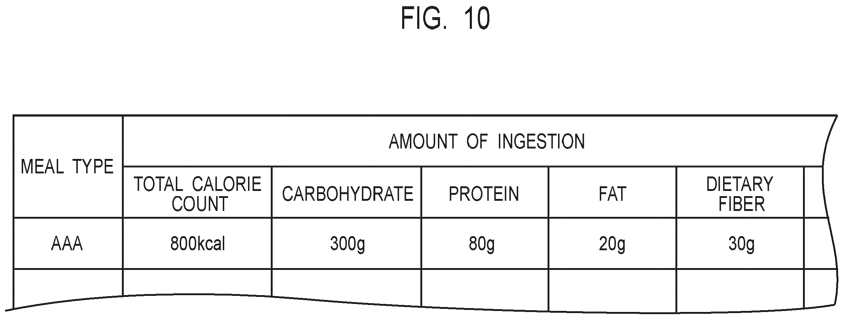

FIG. 10 is a diagram illustrating exemplary nutrition information stored in the information processing device 3 according to a second embodiment. The nutrition database 303 of the information processing device 3 stores nutrition, the contained amount of nutrition, and the total calorie count for each meal type. In the present example, it is indicated for a meal, the meal type of which "AAA" that the total calorie count is "800 kcal", the amount of carbohydrate is "300 g", the amount of protein is "80 g", the amount of fat is "20 g", and the amount of dietary fiber is "30 g". In this manner, since the nutrition information is stored, the contained amount of nutrition and total calorie count of a meal can be specified based on the type of the meal, and the dietary habit of a user can be quantitatively analyzed.

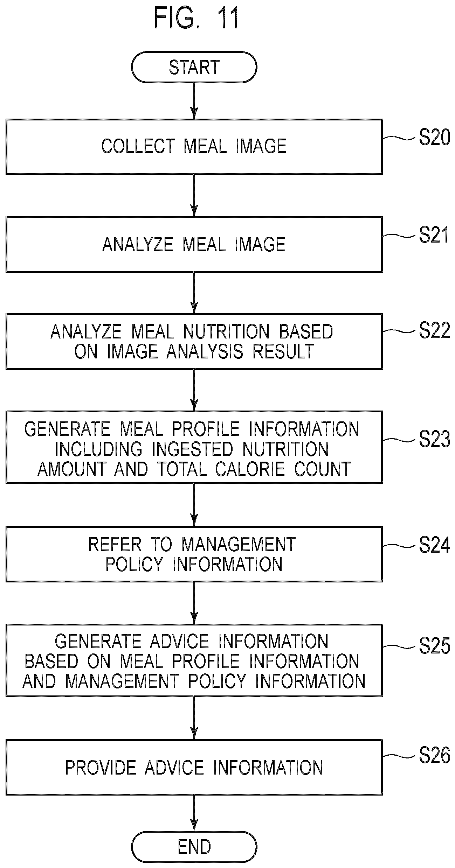

FIG. 11 is a flow diagram of processing executed by the information processing device 3 according to a second embodiment. First, the information processing device 3 collects meal images as action-related information from the first server 2A and the second server 2B (S20), and analyzes the meal images (S21). Then, the information processing device 3 analyzes nutrition of each meal based on a result of the image analysis (S22), and generates meal profile information including the amount of ingested nutrition and the total calorie count (S23). The meal profile information is an exemplary analysis result of the action-related information.

Thereafter, the information processing device 3 refers to management policy information stored in the management policy database 32 (S24), and generates advice information based on the meal profile information and the management policy information (S25).

Lastly, the information processing device 3 provides the generated advice information to an action management service provider having requested provision of the advice information (S26).

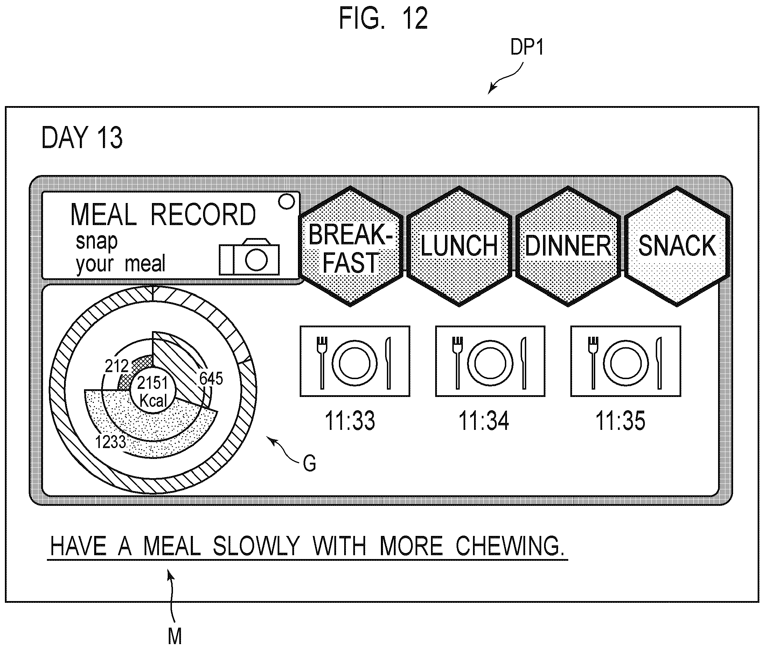

FIG. 12 illustrates an exemplary graphical user interface (GUI) provided to a user terminal 10 by the information processing device 3 according to a second embodiment. The drawing illustrates a first exemplary screen DP1 displayed on a display unit of the user terminal 10. The information processing device 3 according to an embodiment receives action-related information of users from the first user terminal 1A and the second user terminal 1B or from the first server 2A and the second server 2B, and generates target values of tasks related to daily habit improvement based on management policy information corresponding to a type of advice requested by each user among pieces of management policy information. The information processing device 3 may generate target values of tasks related to daily habit improvement based on management policy information corresponding to an action management service provider having requested provision of advice information. The target value of each task is a quantified value of the target of a task for daily habit improvement, and is, for example, a target value of calorie consumption related to an exercise task, or a target value of the amount of ingestion of particular nutrition related to a dieting task. The information processing device 3 transmits the target values of the tasks to the server of the action management service provider having requested provision of advice information, and the target values of the tasks are transmitted from the server to the first user terminal 1A or the second user terminal 1B used by the users. The first user terminal 1A and the second user terminal 1B each display, through the first application 10A and the second application 10B, a GUI including the progress of each task and the ratio of the target value of each task to the sum of the target values of the tasks. FIG. 12 illustrates an exemplary GUI thus displayed on the user terminal 10. The progress of each task indicates how much the target value of the task is achieved. The ratio of the target value of each task to the sum of the target values of the tasks is an amount expressed as Vn/Σₙ₌₁^{N}Vn where N represents the number of kinds of tasks and Vn (n = 1 to N) represents the target value of each task.

The GUI displays "Meal record", and includes thumbnail images of "Breakfast", "Lunch", and "Dinner". Each thumbnail image is displayed with a time at which a meal image was recorded. The GUI may include a message M to the user. In the present example, the message M is "Have a meal slowly with more chewing". In addition, the GUI includes a pie chart G. The pie chart G will be described later in detail with reference to the next drawing.

In this manner, since the information processing device 3 produces target values of tasks related to daily habit improvement in accordance with the subjective need of a user based on management policy information corresponding to a type of advice requested by the user among pieces of management policy information and the information communication terminal of the user displays a GUI including the progress of each task and the ratio of the target value of each task to the sum of the target values, the user can understand the progress and ratio of each task at a glance. In addition, since the information processing device 3 produces target values of tasks related to daily habit improvement based on management policy information corresponding to an action management service provider and the information communication terminal of the user displays a GUI including the progress of each task and the ratio of the target value of each task to the sum of the target values, the user can understand the progress and ratio of each task at a glance.

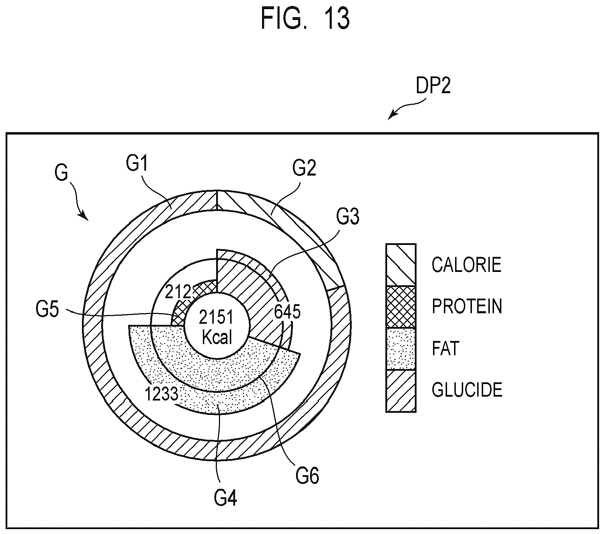

FIG. 13 illustrates an exemplary pie chart G provided to the user terminal 10 by the information processing device 3 according to a second embodiment. The drawing illustrates a second exemplary screen DP2 displayed on the display unit of the user terminal 10. The pie chart G includes the ratio of the target value of each task to the sum of the target values of the tasks. In the present example, the tasks are to bring the amounts of protein, fat, and glucide ingestion per day closer to their target values, and to bring the total calorie ingestion per day closer to its target value. The target values of the tasks include target values related to the amounts of protein, fat, and glucide ingestion per day, and the target value of the total calorie ingestion per day. The ratio of the target value of each task to the sum of the target values of the tasks is displayed in the arc length of a slice in the pie chart G. For example, the ratios of the arc length of glucide G3, the arc length of fat G4, and the arc length of protein G5 indicate the ratios of the ingestion target values of glucide, fat, and protein to the sum of nutrition ingestion target values. In this manner, since the ratio of the target value of each task is displayed in the arc length of a slice in a pie chart, the ratio of the target value of each task to the sum of the target values of the tasks can be understood at a glance as the ratio of the length of the arc. In addition, since the ratio of the target value of each task is displayed in a pie chart, the ratio of the target value of each task to the sum of the target values of the tasks can be understood at a glance.

The pie chart G displays the progress of each task in the width of the pie chart G in the diametrical direction. For example, the width of glucide G3 in the diametrical direction represents the progress of the task of bringing the amount of glucide ingestion per day closer to its target value, the width of fat G4 in the diametrical direction represents the progress of the task of bringing the amount of fat ingestion per day closer to its target value, and the width of protein G5 in the diametrical direction represents the progress of the task of bringing the amount of protein ingestion per day closer to its target value. Since the ratio of the target value of each task is displayed in the arc length of a slice in a pie chart, the ratio of the target value of each task to the sum of the target values of the tasks can be understood at a glance as the ratio of the length of the arc, and since the progress of each task is displayed in the width of a pie chart in the diametrical direction, the progress difference between the tasks can be understood at a glance.

The pie chart G displays the target values of the tasks by a circle G6 concentric with the pie chart G. In the present example, the concentric circle G6 indicates the ratios of the target values of the amounts of protein, fat, and glucide ingestion per day. In the present example, the width of glucide G3 in the diametrical direction slightly exceeds the radius of the concentric circle G6, the width of fat G4 in the diametrical direction largely exceeds the radius of the concentric circle G6, and the width of protein G5 in the diametrical direction is smaller than the radius of the concentric circle G6. Thus, the pie chart G in the present example indicates that the amounts of glucide and fat ingestion are excessive and the amount of protein ingestion is insufficient. In particular, the ratio of the amount of fat ingestion is larger than the ratio of its ingestion target value. In this manner, the target values of the tasks can be understood at a glance while the ratio of the target value of each task to the sum of the target values of the tasks is indicated in the pie chart G through the GUI.

The pie chart G displays the current value or the current total value of any of the tasks by the lengths of arcs G1 and G2 concentric with the pie chart G. In the present example, the pie chart G displays the sum of ingested nutrition calories in the lengths of the arcs G1 and G2 for the task of bringing the total calorie ingestion per day closer to its target value. The arc G1 is an arc of 360° or smaller when the current value of the total calorie count is equal to or smaller than the target value. In other words, the arc G1 becomes a circle when the current value of the total calorie count becomes equal to the target value. The arc G2 indicates an excessive amount of ingestion when the current value of the total calorie count exceeds the target value. In the present example, the length of the arc G2 indicates that the current value of the total calorie count exceeds the target value by 20% approximately. In this manner, the current total value of any of the tasks can be understood at a glance while the ratio of the target value of each task to the sum of the target values of the tasks is indicated in the pie chart through the GUI.

The arcs G1 and G2 concentric with the pie chart G are displayed in a manner different between a case where the sum of the current values of the tasks is smaller than the sum of the target values of the tasks and a case where the sum of the current values of the tasks is larger than the sum of the target values of the tasks. For example, the arc G1 is displayed in green when the sum of the current values of the tasks is smaller than the sum of the target values of the tasks, and the arc G1 is displayed in dark red and the arc G2 indicating the excessive amount is displayed in light red when the sum of the current values of the tasks is larger than the sum of the target values of the tasks. In this manner, since the display is performed in different manners, whether the sum of the current values of the tasks is larger than the sum of the target values can be understood at a glance.

### [Third embodiment]

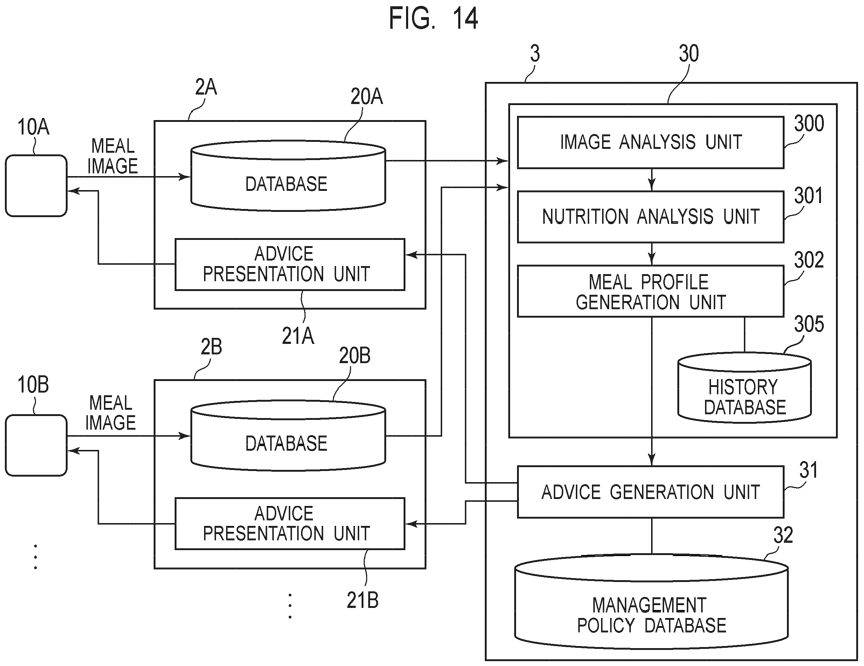

FIG. 14 is a diagram illustrating a functional block of the information processing device 3 according to a third embodiment. Similarly to the information processing device 3 according to a first embodiment, the information processing device 3 according to a third embodiment includes the analysis unit 30, the advice generation unit 31, and the management policy database 32, but is different from the information processing device 3 according to a first embodiment in that the analysis unit 30 includes the image analysis unit 300, the nutrition analysis unit 301, the meal profile generation unit 302, and a history database 305. In an embodiment, the first user and the second user use the first user terminal 1A and the second user terminal 1B to transmit meal images obtained by capturing meals to the first server 2A and the second server 2B, respectively. The first server 2A receives the meal image as action-related information from the first application 10A corresponding to the first user, and stores the meal image in the database 20A. Then, the first server 2A provides the meal image of the first user stored in the database 20A to the analysis unit 30. Similarly, the second server 2B receives the meal image as action-related information from the second application 10B corresponding to the second user, and stores the meal image in the database 20B. Then, the second server 2B provides the action-related information of the second user stored in the database 20B to the analysis unit 30. As similarly to the information processing device 3 according to a second embodiment, the analysis unit 30 includes the nutrition database 303 and the meal profile database 304. The image analysis unit 300, the nutrition analysis unit 301, and the meal profile generation unit 302 have functions same as those in the information processing device 3 according to a second embodiment. In the information processing device 3 according to an embodiment, meal profile information generated by the meal profile generation unit 302 is recorded in the history database 305 in association with a user identifier.

The advice generation unit 31 refers to the history database 305 and suggests advice information again. The advice generation unit 31 generates new advice information based on temporal change of an action of a user having received a service by an action management service provider and management policy information corresponding to an action management service provider having requested advice provision. The temporal change of the action of the user indicates change of a daily habit since the service that manages an action related to the daily habit by the action management service provider is received. More specifically, the temporal change of the action of the user indicates whether the daily habit has improved, has hardly changed, or has degraded since the service by the action management service provider is received. The advice generation unit 31 generates new advice information also when the action of the user having received the service by the action management service provider does not satisfy a criterion requested by advice information. The requested criterion is a criterion as a target related to improvement of the daily habit, and is, for example, to have the amount of exercise equal to or larger than its target value, or to have the amount of ingestion of particular nutrition equal to or smaller than the standard value or equal to or larger than the standard value.

For example, when exceedance of a target value set by advice information has continued for a predetermined duration, the advice generation unit 31 regenerates the advice information. The target value (requested criterion) set by the advice information is, for example, 100 g set as the upper limit of the amount of glucide ingestion per day. In such a case, the advice generation unit 31 generates, for example, advice information of "The amount of glucide ingestion continuously exceeds the target value. Review meal contents.", generates advice information of "The amount of glucide ingestion continuously exceeds the target value. Consult to an adviser.", or generates advice information of "The target of the walking time (30 minutes) per day is continuously achieved. Try setting the target of the walking time per day to 40 minutes.". In this manner, since new advice information is generated in accordance with the change of the action of the user having received a service by an action management service provider, new advice more suitable for the user can be provided based on the effect of the previous advice. In addition, since new advice information is generated when the action of the user having received a service by the action management service provider does not satisfy a requested criterion, new advice more suitable for the user can be provided based on insufficiency of the effect of the previous advice.

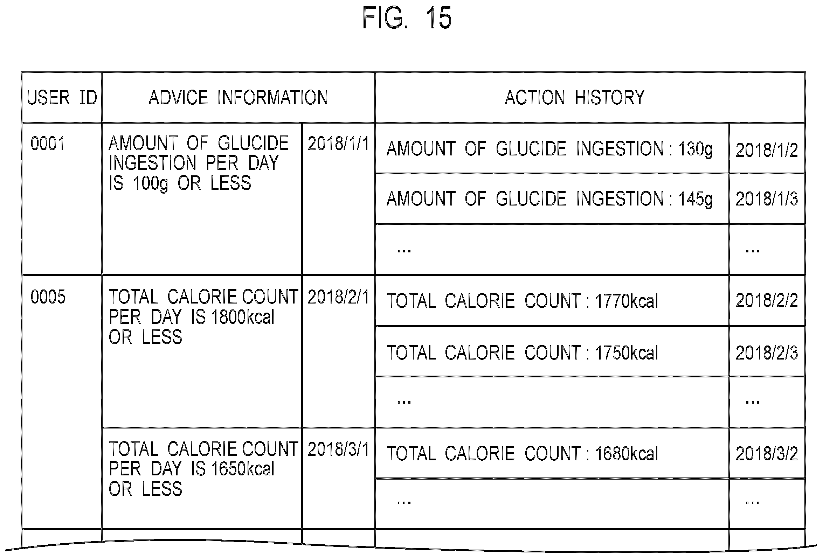

FIG. 15 is a diagram illustrating exemplary history information referred to by the information processing device 3 according to a third embodiment. The history information is stored in the history database 305 for each user ID. In the present example, the history database 305 stores, for each user ID, advice information provided in the past and an action history of the user after the advice information is provided. For example, for a user having the user ID of "0001", the advice information of "the amount of glucide ingestion per day is 100 g or less" was provided on "2018/1/1" (January 1, 2018), and thereafter, the action history of "the amount of glucide ingestion: 130 g" was recorded on "2018/1/2" (January 2, 2018) and the action history of "the amount of glucide ingestion: 145 g" was recorded on "2018/1/3" (January 3, 2018). In addition, for a user having the user ID of "0005", the advice information of "the total calorie count per day is 1800 kcal or less" was provided on "2018/2/1" (February 1, 2018), and thereafter, the action history of "the total calorie count: 1770 kcal" was recorded on "2018/2/2" (February 2, 2018), and the action history of "the total calorie count: 1750 kcal" was recorded on "2018/2/3" (February 3, 2018). In addition, for a user having the user ID of "0005", the advice information of "the total calorie count per day is 1650 kcal or less" was provided on "2018/3/1" (March 1, 2018), and thereafter, the action history of "the total calorie count: 1680 kcal" was recorded on "2018/3/2" (March 2, 2018).

In the present example, the advice generation unit 31 provided the advice information of "the total calorie count per day is 1800 kcal or less" to the user having the user ID "0005" on February 1, 2018, and thereafter, the user had the total calorie count of 1770 kcal or 1750 kcal and achieved the target. Thus, the advice generation unit 31 generated new advice information of "the total calorie count per day is 1650 kcal or less" on March 1, 2018 based on the temporal change of the action of the user that the total calorie count was limited to the target value and management policy information corresponding to an action management service provider. In this manner, when the user has achieved a target, the advice generation unit 31 newly generates advice information in accordance with a higher target. In addition, when the user has not achieved the target, the advice generation unit 31 newly generates advice information in accordance with a target that can be achieved more easily.

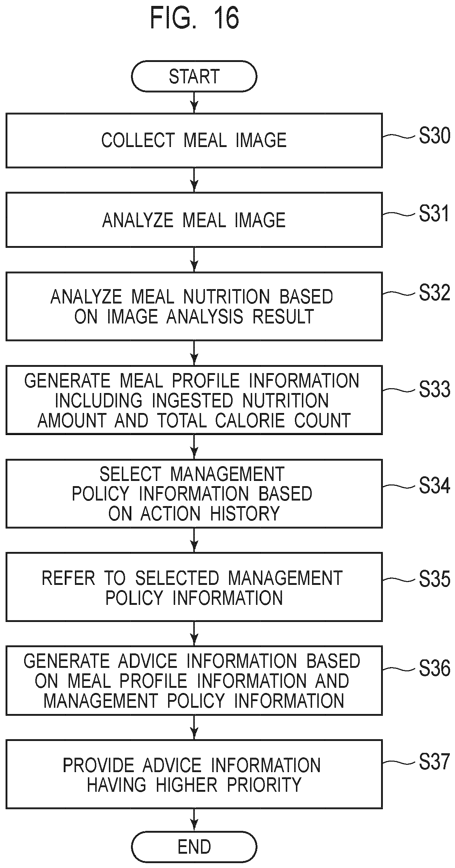

FIG. 16 is a flow diagram of processing executed by the information processing device 3 according to a third embodiment. First, the information processing device 3 collects meal images as action-related information from the first server 2A and the second server 2B (S30), and analyzes the meal images (S31). Then, the information processing device 3 analyzes the nutrition of each meal based on a result of the image analysis (S32), and generates meal profile information including the amount of nutrition ingestion and the total calorie count (S33).

Thereafter, the information processing device 3 extracts the action history of the user from the history database 305, selects management policy information stored in the management policy database 32 based on the action history (S34), and refers to the management policy information (S35). Then, the information processing device 3 generates advice information based on the meal profile information and the management policy information, (S36). The information processing device 3 may generate a plurality of pieces of advice information.

Lastly, the information processing device 3 provides advice information having a higher priority among the generated pieces of advice information to an action management service provider having requested provision of advice information (S37).

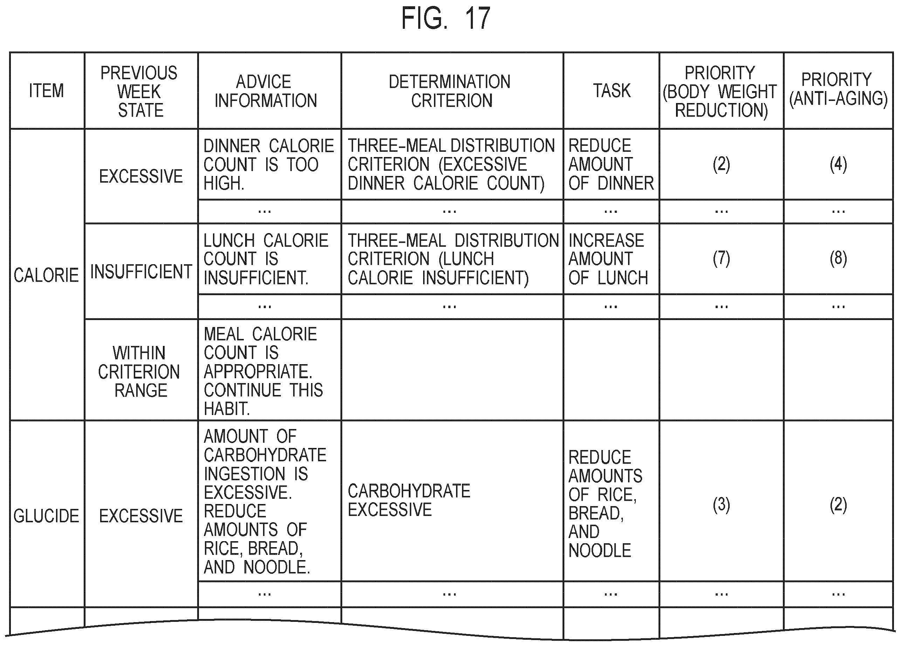

FIG. 17 is a diagram illustrating exemplary advice information generated by the information processing device 3 according to a third embodiment. In the present example, new advice information, determination criterion, task information, and priority for an action of a user a week before are provided for each item of "calorie" or "glucide".

For example, the item of "calorie" has the three states of "excessive", "insufficient", and "within criterion range" for "previous week state". Specifically, when management policy information is to bring the ingestion calorie count closer to its target value, the item has a state indicating whether the user did not achieve its target and the calorie count was excessive or insufficient, or whether the user achieved the target. When "previous week state" is "within criterion range", advice information is "The meal calorie count is appropriate. Continue this habit.".

When "previous week state" is "excessive", the advice information is "The dinner calorie count is too high.". Such advice information is generated when "three-meal distribution criterion (excessive dinner calorie count)" as "determination criterion" is satisfied. Specifically, advice information indicating that the dinner calorie count is high is generated when the dinner calorie count exceeds the target value in calorie distribution of the three meals of breakfast, lunch, and dinner. Advice information indicating exceedance may be generated when the breakfast and lunch calorie counts are excessive, and advice information indicating exceedance may be generated may be generated when the snack calorie count is excessive.

A task when the dinner calorie count exceeds is to "reduce the amount dinner". The task is a specific challenge to be performed by the user, and advice information may be information prompting the user to recognize the current state.

When the dinner calorie count exceeds, the priority in which the advice information is provided is "2" for "priority (body weight reduction)" and "4" for "priority (anti-aging)". "Body weight reduction" and "anti-aging" are exemplary management policies or management policy information, which indicates difference in the purpose of limiting the ingestion calorie count. For example, when the management policy information is body weight reduction, it is thought that the exceedance of the dinner calorie count interferes with target achievement, and the priority in which the advice information is provided is set to the second place. When the management policy information is anti-aging, it is thought that the exceedance of the dinner calorie count provides relatively small influence on target achievement, and the priority in which the advice information is provided is set to the fourth place.

When "previous week state" is "insufficient", the advice information is "The lunch calorie count is insufficient.". Such advice information is generated when "three-meal distribution criterion (lunch calorie insufficient)" as "determination criterion" is satisfied. Specifically, advice information indicating the insufficiency of the lunch calorie count is generated when the lunch calorie count is smaller than its target value in calorie distribution of the three meals of breakfast, lunch, and dinner. Advice information indicating insufficiency may be generated when the breakfast and dinner calorie counts are insufficient.

A task when the lunch calorie count is insufficient is to "increase the amount of lunch". When the lunch calorie count is insufficient, the priority in which the advice information is presented is "7" for "priority (body weight reduction)", and "8" for "priority (anti-aging)". For example, when management policy information is body weight reduction, it is thought that the insufficiency of the lunch calorie count does not interfere with target achievement, and the priority in which the advice information is provided is set to the seventh place. When management policy information is anti-aging, it is thought that the insufficiency of the lunch calorie count does not interfere with target achievement, and the priority in which the advice information is provided is set to the eighth place.

The item of "glucide" includes "excessive" as "previous week state". However, the item of "glucide" may include the three states of "excessive", "insufficient", and "within criterion range" as "previous week state". Specifically, when management policy information is to bring the amount of glucide ingestion closer to its target value, the item may include a state indicating whether the user did not achieve the target and the amount of glucide ingestion was excessive or insufficient or whether the user achieved the target.

When "previous week state" is "excessive", the advice information is "The amount of carbohydrate ingestion is excessive. Reduce the amounts of rice, bread, and noodle.". Such advice information is generated when "carbohydrate excessive" as "determination criterion" is satisfied. Specifically, when the amount of carbohydrate ingestion at a meal exceeds its target value, advice information indicating the exceedance of the amount of carbohydrate ingestion is generated.

A task when carbohydrate is excessive is to "reduce the amounts of rice, bread, and noodle". When carbohydrate is excessive, the priority in which the advice information is presented is "3" for "priority (body weight reduction)", and "2" for "priority (anti-aging)". For example, when management policy information is body weight reduction, it is thought that excessive carbohydrate interferes with target achievement, and the priority in which the advice information is provided is set to the third place. When management policy information is anti-aging, it is thought that carbohydrate excessive interferes with target achievement, and the priority in which the advice information is provided is set to the second place.

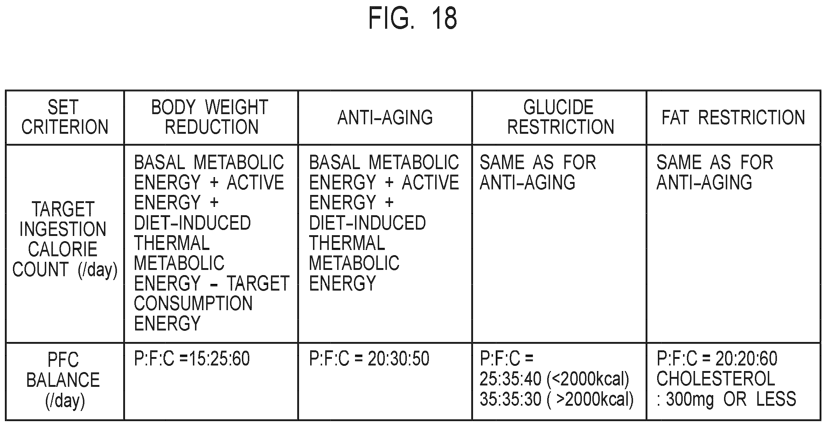

FIG. 18 is a diagram illustrating exemplary set target values referred to by the information processing device 3 according to a third embodiment. In the present example, the target ingestion calorie per day and FPC (fat, protein, carbohydrate) balance are exemplarily set for each of the four management policies of "body weight reduction", "anti-aging", "glucide restriction", and "fat restriction". The FPC balance means the balance among fat, protein, and carbohydrate (glucide).

In the present example, "target ingestion calorie count (/day)" is "basal metabolic energy + active energy + diet-induced thermal metabolic energy - target consumption energy" when management policy information is "body weight reduction". Specifically, the ingestion calorie count per day is a value obtained by subtracting target consumption energy consumed through exercise from the sum of calories consumed through basal metabolism, active energy consumed through daily life, and diet-induced thermal metabolic calories consumed through dieting. When such a target ingestion calorie count is set, the calorie balance per day decreases by the energy consumed through exercise, which leads to body weight reduction.

In the present example, "target ingestion calorie count (/day)" is "basal metabolic energy + active energy + diet-induced thermal metabolic energy" when management policy information is "anti-aging". Specifically, the ingestion calorie count per day is the sum of calories consumed through basal metabolism, active energy consumed through daily life, and diet-induced thermal metabolic calories consumed through dieting. When such a target ingestion calorie count is set, the calorie balance per day can be made zero. When management policy information is "glucide restriction" or "fat restriction", "target ingestion calorie count (/day)" is "same as for anti-aging".

In the present example, "FPC balance (/day)" is "P:F:C = 15:25:60" when management policy information is "body weight reduction". Specifically, with the balance, protein, fat, and carbohydrate are ingested at the ratio of 15:20:60. When the value of the target ingestion calorie count is set, the target ingestion calorie count of each of protein, fat, and carbohydrate can be calculated based on the FPC balance.

"FPC balance (/day)" is "P:F:C = 20:30:50" when management policy information is "anti-aging". When management policy information is "glucide restriction", "FPC balance (/day)" is "P:F:C = 25:35:40" for a target ingestion calorie smaller than 2000 kcal, or is "P:F:C =35:35:30" for a target ingestion calorie larger than 2000 kcal. When management policy information is "fat restriction", "FPC balance (/day)" is "P:F:C = 20:20:60" with an additional limit of "cholesterol: 300 mg or less".

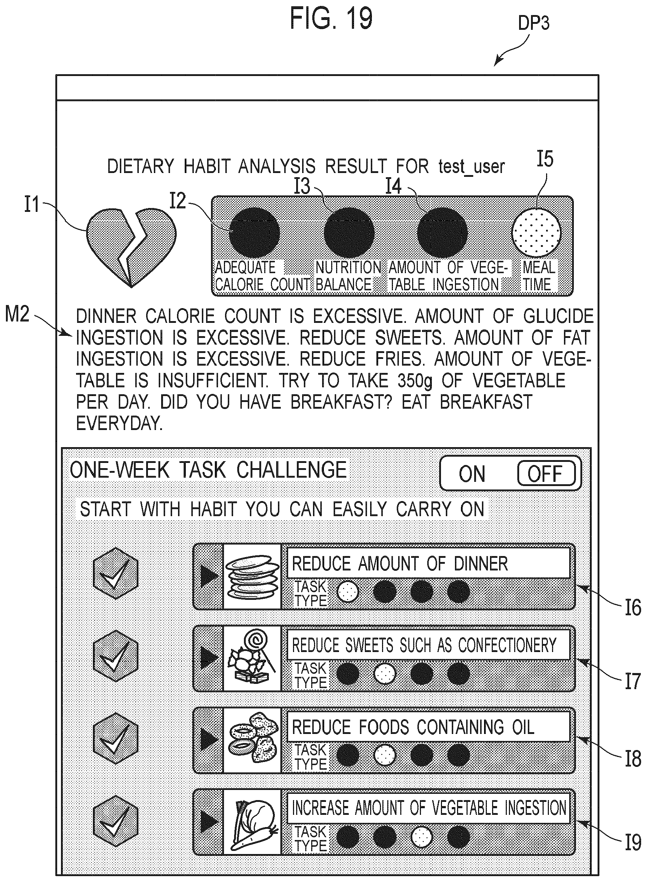

FIG. 19 illustrates a first exemplary GUI provided to the user terminal 10 by the information processing device 3 according to a third embodiment. The drawing illustrates a third exemplary screen DP3 displayed on the display unit of the user terminal 10. The third exemplary screen DP3 is displayed, for example, after meal images for a week are recorded and the action history of the user is accumulated.

A first icon I1 included in the third exemplary screen DP3 indicates whether the user has achieved tasks. In the present example, the first icon I1 has a cracked heart shape, which indicates that at least any one of the tasks has not been achieved. In the present example, the tasks are to bring the total calorie count closer to its target value, to bring the nutritional balance closer to its target value, to bring the amount of vegetable ingestion to its target value or more, and to bring each meal time closer to its target value.

A second icon I2 indicates whether the total calorie count has been brought closer to its target value. In the present example, the total calorie count has not been brought closer to its target value, and thus the second icon I2 is displayed in red. The second icon I2 is displayed in green when the tasks have been achieved, in yellow when not all but some tasks have been achieved, or in red when the tasks have not been achieved.

A third icon I3 indicates whether the nutritional balance has been brought closer to its target value. In the present example, the nutritional balance has not brought closer to its target value, and thus the third icon I3 is displayed in red. A fourth icon I4 indicates whether the amount of vegetable ingestion has been brought to its target value or more. In the present example, the amount of vegetable ingestion has not been brought to the target value or more, and thus the fourth icon 14 is displayed in red. A fifth icon I5 indicates whether each meal time has been brought closer to its target value. In the present example, the meal time has been brought closer to its target value, and thus the fifth icon I5 is displayed in green. In this manner, since each icon is displayed in different manners in accordance with the achievement rate of the corresponding task, which of the tasks has not been achieved can be understood at a glance.

A second message M2 included in the third exemplary screen DP3 includes advice information generated by the advice generation unit 31. The advice generation unit 31 generates pieces of advice information based on an analysis result of action-related information of the user and management policy information, and provides advice information having a higher priority to the user terminal 10 through the first server 2A and the second server 2B. In the present example, the second message M2 is "The dinner calorie count is excessive. The amount of glucide ingestion is excessive. Reduce sweets. The amount of fat ingestion is excessive. Reduce fries. The amount of vegetable is insufficient. Try to take 350 g of vegetable per day. Had breakfast? Eat daily.". This advice information is advice information generated based on such action-related information of the user that the tasks of bringing the total calorie count closer to its target value, bringing the nutritional balance closer to its target value, and bringing the amount of vegetable ingestion to its target value or more have not achieved and the management policy information.

The third exemplary screen DP3 also includes "one-week task challenge". Items included in "one-week task challenge" are items related to tasks generated when the action of the user having received a service by the action management service provider does not satisfy a criterion requested by the advice information. In the present example, a sixth icon I6 indicates the task of "Reduce the amount of dinner". A "task type" of the sixth icon I6 is a first type (leftmost type among the four items arranged in a horizontal line). Each "task type" corresponds to the position of the second icon I2, the third icon I3, the fourth icon I4, or the fifth icon I5, and indicates whether the corresponding task is to bring the total calorie count closer to its target value, to bring the nutritional balance closer to its target value, to bring the amount of vegetable ingestion to its target value or more, or to bring each meal time closer to its target value.

A seventh icon I7 indicates the task of "reduce sweets such as confectionary". The "task type" of the seventh icon I7 is a second type (second leftmost type among the four items arranged in a horizontal line). An eighth icon I8 indicates the task of "reduce foods containing oil". The "task type" of the eighth icon I8 is the second type (second leftmost type among the four items arranged in a horizontal line). In addition, a ninth icon I9 indicates the task of "increase the amount of vegetable ingestion". The "task type" of the ninth icon I9 is a third type (third leftmost type among the four items arranged in a horizontal line).

The user freely selects the tasks indicated as "one-week task challenge". In the present example, the all of four tasks are selected and checked. The user may not select a presented task. In this manner, when the user is provided with the freedom of selection and then works on a selected task, the achievement rate and persistence rate of a task can be improved as compared to a case in which the task is simply presented.

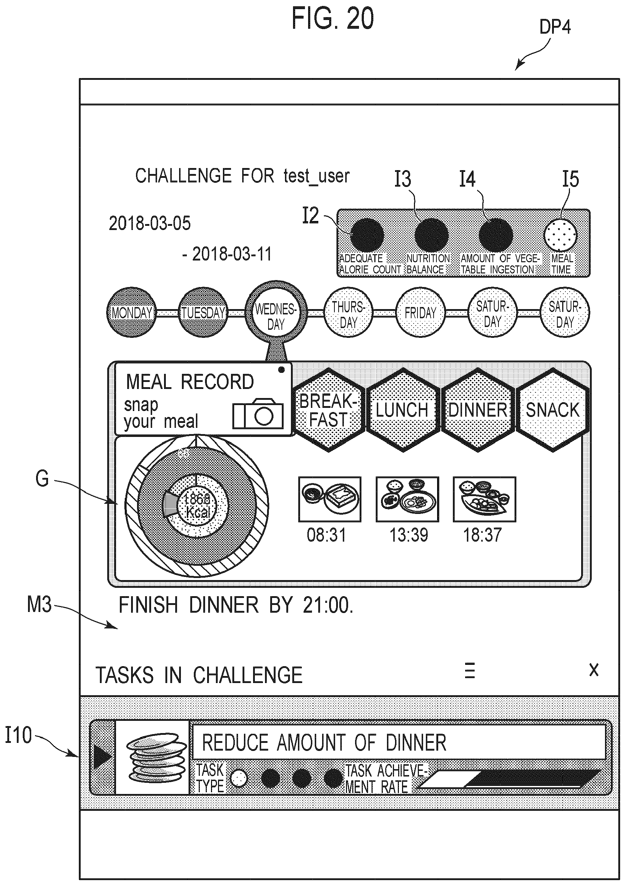

FIG. 20 illustrates a second exemplary GUI provided to the user terminal 10 by the information processing device 3 according to a third embodiment. The drawing illustrates a fourth exemplary screen DP4 displayed on the display unit of the user terminal 10. The fourth exemplary screen DP4 is displayed on the user terminal 10 while the "one-week task challenge" illustrated in FIG. 19 is being worked on. In the present example, records for the week of "2018-03-05" (March 5, 2018) to "2018-03-11" (March 11, 2018) are displayed.

The second icon I2, the third icon I3, the fourth icon I4, and the fifth icon I5 included in the fourth exemplary screen DP4 indicate the achievement rates of the tasks. The second icon I2, the third icon I3, and the fourth icon I4 are displayed in red, indicating that the tasks of bringing the total calorie count closer to its target value, bringing the nutritional balance closer to its target value, and bringing the amount of vegetable ingestion to its target value or more have not been achieved in the previous week. The fifth icon I5 is displayed in green, indicating that the task of bringing each meal time closer to its target value has been achieved in the previous week.

The pie chart G is same as that illustrated in FIG. 13, and illustrates the progress of each task and the ratio of the target value of each task to the sum of the target values of the tasks. "Meal record" displays, with record times, thumbnail images obtained by capturing breakfast, lunch, and dinner on Wednesday (Wednesday March 7, 2018). In the present example, the breakfast was recorded at "08:31" (8:31 a.m.), the lunch was recorded at "13:39" (1:39 p.m.), and the dinner was recorded at "18:37" (6:37 p.m.).

A third message M3 is "Finish dinner by 21:00.", and is a message related to the task of bringing each meal time closer to its target value. A tenth icon I10 illustrates "tasks in challenge", and in the present example, the task of "have breakfast" is illustrated. The "task type" of the tenth icon I10 is the first type (leftmost type among the four items arranged in a horizontal line). "Task achievement rate" displays, in a bar, the number of days on which any task is achieved in a week in such a manner that the bar extends by a piece as the number of days on which any task is achieved increases by one, and the bar is filled when tasks are consecutively achieved for a week. Accordingly, the achievement rate of tasks can be understood at a glance.

The embodiments described above are intended to facilitate understanding of the present invention but not to limit the present invention. Elements included in the embodiments and their disposition, materials, conditions, shapes, sizes, and the like are not limited to those exemplarily described above but may be changed as appropriate. Components described in different embodiments may be partially interchanged or combined.

## Claims

1. A computer-implemented method of generating advice information concerning a daily habit, the method comprising:
analyzing action-related information on an action of a user to prepare an analysis result; and
generating advice information concerning improvement of a daily habit of the user based on:
the analysis result; and
a piece of management policy information corresponding to a type of advice requested by the user among pieces of management policy information.

2. The method according to claim 1, wherein:
generating the advice information comprises generating the advice information based on:
the analysis result; and
the piece of management policy information selected from among the pieces of management policy information based on predetermined identification information.

3. The method according to claim 1 or 2, wherein
analyzing the action-related information comprises extracting a characteristic of the daily habit of the user as the analysis result, and
generating the advice information comprises generating the advice information based on:
the extracted characteristic of the daily habit as the analysis result; and
the piece of management policy information.

4. The method according to anyone of claims 1 to 3, further comprising:
adjusting the piece of management policy information based on a profile of the user.

5. The method according to any one of claims 1 to 4, wherein
generating the advice information comprises generating, in response to a request from an action management service provider that provides a service to manage an action related to the daily habit of the user to the user, the advice information based on:
the analysis result; and
a piece of management policy information corresponding to the action management service provider among the pieces of management policy information, wherein
the method further comprises providing the generated advice information to the action management service provider having made the request.

6. The method according to claim 5, wherein
generating the advice information comprises generating the advice information based on:
the analysis result; and
the piece of management policy information corresponding to the action management service provider selected based on identification information of the action management service provider.

7. The method according to claim 5 or 6, further comprising:
generating task information supporting the user receiving the service provided by the action management service provider.

8. The method according to any one of claims 5 to 7, further comprising:
generating new advice information concerning improvement of the daily habit based on:
temporal change of an action of the user having received the service provided by the action management service provider; and
the piece of management policy information corresponding to the action management service provider having made the request.

9. The method according to claim 8, wherein
generating the new advice information comprises generating the new advice information in response to the action of the user having received the service provided by the action management service provider not satisfying a criterion requested by the advice information.

10. The method according to any one of claims 5 to 9, wherein
the action management service provider comprises a plurality of action management service providers, and
the plurality of action management service providers provide services to manage actions related to daily habits to a plurality of users.

11. The method according to any one of claims 5 to 10, wherein
the action management service provider comprises two or more action management service providers, and
generating the advice information comprises generating two or more different pieces of advice information corresponding to the two or more action management service providers having made requests based on:
the analysis result; and
two or more pieces of management policy information corresponding to the two or more action management service providers.

12. The method according to any one of claims 1 to 11, wherein the action-related information comprises at least one of a meal image obtained by capturing a meal of the user, vital data of the user, and information indicating an amount of exercise of the user.

13. The method according to any one of claims 1 to 12, further comprising:
providing the advice information to a computer that communicates with an application installed on a terminal for the user.

14. The method according to any one of claims 1 to 13, further comprising:
providing the advice information through an application program interface (API) to a computer that communicates with an application installed on a terminal for the user.

15. An information processing device that generates advice information concerning a daily habit, the information processing device comprising:
an analysis unit that analyzes action-related information on an action of a user to prepare an analysis result; and
an advice generation unit that generates advice information concerning improvement of a daily habit of the user based on:
the analysis result; and
a piece of management policy information corresponding to a type of advice requested by the user among pieces of management policy information.
